Europäisches Patentamt

European Patent Office

Office européen des brevets

(11) Publication number: **0 589 876 B1**

# EUROPEAN PATENT SPECIFICATION

(45) Date of publication of patent specification: **29.11.95**

(51) Int. Cl.6: **C07C 15/02**, C07C 2/66, C07C 6/12

(21) Application number: **91912754.8**

(22) Date of filing: **21.06.91**

(86) International application number:
**PCT/US91/04418**

(87) International publication number:
**WO 93/00317 (07.01.93 93/02)**

(54) **ALKYLATION OF AROMATIC COMPOUNDS.**

(43) Date of publication of application:
**06.04.94 Bulletin 94/14**

(45) Publication of the grant of the patent:
**29.11.95 Bulletin 95/48**

(84) Designated Contracting States:
**AT BE CH DE DK ES FR GB GR IT LI LU NL SE**

(56) References cited:
**EP-A- 0 317 907
EP-A- 0 402 202
EP-A- 0 433 932
WO-A-89/10910
FR-A- 2 084 704**

(73) Proprietor: **THE DOW CHEMICAL COMPANY
2030 Dow Center,
Abbott Road
Midland,
Michigan 48640 (US)**

Proprietor: **DOW BENELUX N.V.
Herbert H. Dowweg 5
NL-4542 NM Hoek (NL)**

(72) Inventor: **LEE, Guo, Shuh, John
10 Burrell Court
Midland, MI 48640 (US)**
Inventor: **GARCES, Juan, M.
1106 West Sugnet
Midland, MI 48640 (US)**
Inventor: **MEIMA, Garmt, R.
12 Amperestraat
NL-4532 LN Terneuzen (NL)**
Inventor: **VAN DER AALST, Matheus, J., M.
36 Dinkelstraat
NL-4535 EC Terneuzen (NL)**

(74) Representative: **Smulders, Theodorus A.H.J.,
Ir. et al
Vereenigde Octrooibureaux
Nieuwe Parklaan 97
NL-2587 BN 's-Gravenhage (NL)**

## Description

This invention relates to a process of alkylating benzene or substituted benzene with an alkylating agent having from two to eighteen carbon atoms using acidic mordenite zeolites as catalyst to produce aromatic compounds such as cumene, ethylbenzene and other alkylated benzenes.

Cumene, also known as isopropylbenzene, is useful for the production of phenol, acetone and alpha-methylstyrene. Ethylbenzene is useful in the production of styrene. Various processes for their manufacture are known.

Various processing schemes comprising alkylation and/or transalkylation are known to produce monoalkylaromatic products such as cumene or ethylbenzene in high yields. Up to now, basically only two types of processes are used in commercial practice; one of which is a liquid phase process using catalysts such as solid phosphoric acid or Friedel Crafts catalysts such as aluminum chloride or boron fluoride, the other one being a gas phase process using zeolite type catalysts. A major drawback of the liquid phase type processes is their negative impact on the environment, the solid catalysts being not regenerable and thus are discarded as waste continuously or batchwise depending on the particular process. A further drawback associated for example with the use of phosphoric acid catalysts is that generally the use of a water co-feed is required which produces a corrosive sludge byproduct. Major drawbacks of the zeolite catalyzed gas phase processes are the production of undesirable by-products and the relatively rapid deactivation of the catalyst. Examples of such byproducts produced in conjunction with cumene include: alkylating agent oligomers, heavy polyaromatic compounds and unwanted monoalkylated and dialkylated compounds such as n-propylbenzene, butylbenzenes and ethylbenzene; and in conjunction with ethylbenzene: the production of alkylating agent oligomers, heavy polyaromatic compounds, unwanted xylenes, toluene, butylbenzenes, cumene, and n-propyl benzene.

It is known that aromatic hydrocarbons can be alkylated in the presence of acid-treated zeolite. U.S. Patent 4,393,262 (1983) teaches that cumene is prepared by the alkylation of benzene with propylene in the presence of a specified zeolite catalyst. U.S. Patent 3,140,253 (1964) and U.S. Patent 3,367,884 (1968) broadly teach the use of acid-treated mordenite for the alkylation of aromatic compounds. However, such alkylations are generally not selective with respect to the number of substitutions. Further, catalysts are often quickly deactivated requiring timely and costly replacements or reactivation.

European Patent Application No. 0366515 teaches a process for the production of at least an alkyl benzene wherein benzene is reacted with a charge containing at least an aliphatic mono-olefin in the presence of a catalyst which is based on a dealuminated mordenite having a Si/Al atomic ratio between from 30 to 80 (equal to a silica/alumina molar ratio of 60-160). At Si/Al atomic ratios higher than 80 (i.e. silica/alumina molar ratio higher than 160) the catalyst activity is decreased, a conversion less than substantially 100 percent is achieved and the catalyst deactivates rapidly (see Example 2A). The process may be performed in liquid, supercritical or gas phase over a fixed bed of catalyst. This reference further teaches a transalkylation step following the above-described alkylation step in order to optimize the overall yield of mono-alkylated benzenes, reacting a polyalkyl benzene fraction obtained in the alkylation step with benzene using a catalyst which is based on a dealuminated mordenite having a Si/Al atomic ratio between from 30 to 80 (equal to a silica/alumina molar ratio of 60-160).

European Patent Application No. 0402202 teaches substantially the same alkylation/transalkylation process as EP-0366515, only replacing in the alkylation step the dealuminated mordenite catalyst by a dealuminated Y zeolite catalyst having a silica/alumina molar ratio of between 8 and 70. By doing so it desires to improve the alkylation yield to mono-alkyl benzenes by reducing the proportion of polyalkyl benzenes formed.

Important criteria which determine the feasibility of a process for the alkylation of benzene or substituted benzene, besides the above-mentioned criteria regarding environmental impact, low level of impurities which are difficult to remove from the process stream and/or to convert to desired products, activity and stability of the catalyst (decreased deactivation), include the following: the conversion based on the alkylating agent should be substantially 100 percent in order to prevent expensive recycling of unconverted alkylating agent or loss of alkylating agent to flare; the selectivity towards the mono-alkylated benzene should be high; and the recycle of benzene or substituted benzene should be minimized as it is rather expensive and leads to larger quantities of impurities which are recycled to the alkylation reactor and passed over the catalyst. Minimizing the recycle of benzene or substituted benzene could be achieved by decreasing the molar ratio of benzene or substituted benzene to alkylating agent. However, it is known that the conventional zeolite type catalysts tend to deactivate under these conditions because of increased tendency of the alkylating agent to polymerize and because of the increased polyalkylation activity. Apart from catalyst deactivation this, moreover, would lead to a decreased selectivity towards the mono-alkylated benzene. Therefore, the main object of the conventional zeolite catalyzed processes was to optimize conversion of the alkylating agent and selectivity towards the mono-alkylated product in the alkylation step or process in itself.

Surprisingly, it has now been found that by using a specific mordenite catalyst in combination with a relatively low molar ratio of benzene or substituted benzene to alkylating agent in a liquid phase process, an alkylation process is obtained having a low environmental impact, low levels of impurities which are difficult to remove from the process stream and/or difficult to convert to desired products, highly stable catalyst, substantially 100 percent conversion of the alkylating agent, low benzene or substituted benzene recycle, a high selectivity towards the total of mono-alkylated products and dialkylated products, and a low selectivity towards the tri- and tetra-alkylated products. This alkylation process is very suitable as a first step in an overall alkylation/transalkylation process to produce mono-alkylated benzenes or mono-alkylated substituted benzenes in a very high yield.

Accordingly, the present invention provides a process of alkylating benzene or substituted benzene comprising contacting the benzene or substituted benzene with an alkylating agent having from two to eighteen carbon atoms in the presence of a catalyst comprising an acidic mordenite zeolite having a silica/alumina molar ratio of higher than 160:1 and a crystalline structure which is determined by X-ray diffraction to have a Symmetry Index of at least 1, under temperature and pressure conditions sufficient to keep the reaction mixture in the liquid phase, and the molar ratio of benzene or substituted benzene to alkylating agent is less than 3:1 and greater than 1:1.

The alkylation process according to the present invention provides a well-balanced combination of low levels of undesired byproducts, a liquid phase process with relatively mild operating conditions, an active catalyst which provides at the low benzene or substituted benzene to alkylating agent molar ratios a conversion of the alkylating agent of substantially 100 percent, and a high selectivity towards monoalkylated benzene and polyalkylated benzenes having the same alkyl substituent as in the desired monoalkylated product. The major product is the monoalkylated product, whereas the polyalkylated benzenes are primarily dialkylated benzenes which may be easily converted in an optional subsequent transalkylation step. These advantages are achieved under conditions of low benzene or substituted benzene to alkylating agent ratios and substantially without increasing catalyst deactivation rate, which is very surprising in view of the prior art.

Any monocyclic aromatic compound may be alkylated by the process of this invention. The aromatic compound is preferably benzene or substituted benzene. Non-limiting examples of substituted benzenes which may be alkylated by the process of this invention include toluene, xylenes, phenol and aniline. In a preferred embodiment, benzene is the aromatic compound which is alkylated.

The aromatic compound may be used neat in a liquid state, or dissolved in a suitable solvent. Preferably, the aromatic compound is used in a neat liquid state. If a solvent is employed, any inert solvent which solubilizes the aromatic compound and does not hinder the alkylation reaction may be used. The preferred solvent is a saturated hydrocarbon.

The alkylating agent used may be any aliphatic or cycloaliphatic olefin having from two to eighteen carbon atoms. Preferably an aliphatic olefin having from two to twelve carbon atoms is used and more preferably ethylene or propylene.

The catalyst useful in the practice of this invention comprises an acidic mordenite zeolite having a silica/alumina molar ratio of at least 160:1, a Symmetry Index (SI) as defined hereinafter of at least about 1.0.

The catalyst useful in this invention can be prepared by a process which comprises dealumination of a mordenite zeolite having a silica/alumina molar ratio less than 30:1 and a crystalline structure which is determined by X-ray diffraction to possess a Symmetry Index (SI) of from about 0.5 to about 1.3 and more preferably of from about 0.7 to about 1.3 under conditions sufficient to remove an amount of alumina sufficient to provide a silica/alumina molar ratio of at least 160:1.

Natural mordenite is an aluminosilicate whose typical unit cell contents are assigned the formula $Na_8$ $[(AlO_2)_8 (SiO_2)_{40} \cdot 24\ H_2O]$. Mordenite is the most siliceous natural zeolite with a silicon/aluminum mole ratio (Si/Al) of about 5/1. It has interconnecting twelve-ring and eight-ring channels. The dimensions of the twelve-ring pores are about $6.7 \times 7.0$ Å; the dimensions of the eight-ring pores are about $2.9 \times 5.7$ Å. The structure and properties of mordenite zeolite are described in Zeolite Molecular Sieves, by Donald W. Breck (John Wiley & Sons, 1974), at pages 122-124 and 162-163, which is incorporated herein by reference.

The mordenite zeolite used to prepare the catalyst of this invention may be selected from a mordenite zeolite typically containing cations of the alkali or alkaline earth metals, or alternatively ammonium ions. Depending upon the source of the raw materials employed in preparing the starting mordenite, the latter may contain varying amounts of metal ions other than the metal ions mentioned above. Mordenites prepared from clays, for example, may contain significant amounts of iron, lesser amounts of cobalt, copper and nickel, and even lesser amounts of other transition elements and rare earths. Mordenites prepared from fumed silica, however, may contain only trace amounts of these extraneous metals, since fumed silica is generally quite pure. The metal ions of the above-mentioned metals, such as sodium, magnesium or calcium, which are often present in starting mordenite samples, may be present in trace amounts in the acidic mordenite catalyst of this invention. Preferably, the acidic mordenite zeolite material for the catalyst of the invention is prepared from a sodium mordenite zeolite; even more preferably, from a sodium mordenite zeolite having a Symmetry Index of from about 0.7 to about 1.3. The Symmetry Index is a dimensionless number obtained from the X-ray diffraction pattern of the sodium mordenite being measured in the hydrated form. Standard techniques are employed to obtain the X-ray data. The radiation is the $K\alpha_1$ line of copper, and a Philips Electronics spectrometer is used. The mordenite zeolites exhibit an X-ray diffraction pattern whose diffraction peaks have d-spacings corresponding to those of crystalline mordenites as reported by J. D. Sherman and J. M. Bennett in "Framework Structures Related to the Zeolite Mordenite," Molecular Sieves; J.W. Meier and J.B. Uytterhoeven, eds., Advances in Chemistry Series, 121, 1973, pp. 52-65. The Symmetry Index is defined as the sum of the peak heights of the [111] (13.45, $2\theta$) and [241] (23.17 $2\theta$) reflections divided by the peak height of the [350] (26.25 $2\theta$) reflection.

Four ordered crystalline structures have been proposed to describe the X-ray diffraction data available for natural and synthetic mordenites. (J. D. Sherman and J. M. Bennett, op.cit., p. 53.) The symmetries of these four structures are Cmcm, Cmmm, Imcm, and Immm as these terms are defined by N. F. M. Henry and K. Lonsdale in International Tables for X-ray Crystallography, 3rd Ed., Volume 1, Kynoch Press (1969). X-ray diffraction data indicate that mordenites are either physical admixtures or intergrowths of the Cmmm, Imcm, or Immm structures with the Cmcm structure. Thus, mordenites can be generally described as having a crystalline structure comprising a matrix of Cmcm symmetry having dispersed therein domains of Cmmm, Imcm, or Immm symmetry, or mixtures thereof. Preferably, the mordenite starting material has a crystalline structure comprising a matrix of Cmcm symmetry having dispersed therein domains of Cmmm symmetry. The Symmetry Index is related to the symmetries of the crystals present in the mordenite sample. A Symmetry Index in the range from about 0.70 to about 1.3 provides the optimum sodium mordenite as starting material for the process of this invention.

The crystallite size of the original sodium mordenite may be any size, but typically the crystallite size may be in the range from about 500 Å to about 5000 Å. Preferably, the crystallite size is in the range from about 500 Å to about 2000 Å; more preferably, from about 800 Å to about 1500 Å. Generally, the crystallites form aggregates which may be used as such or bound into larger particles for the preparation of the catalyst to be used in the process of this invention.

The original sodium mordenite zeolite described hereinabove, or its equivalent, is treated to obtain the catalyst of the invention for use in the alkylation process. Intergrowths of mordenite zeolite with other zeolites are also suitable starting materials. The treatment involves contacting the mordenite with acid. In one preferred embodiment, the treatment involves contacting the mordenite with acid, calcining the acid-treated mordenite, and further contacting the calcined mordenite with strong acid. In an alternative preferred embodiment, the catalyst is prepared without being calcined.

The initial acid treatment serves to remove most of the sodium ions, or their equivalents, from the original mordenite. The treatment may remove a portion of the aluminum ions as well. Inorganic acids and organic acids are suitable compounds from which the hydrogen ions are obtained for the acid treatment. Examples of such acids are hydrochloric acid, hydrobromic acid, sulfuric acid, phosphoric acid, nitric acid, acetic acid, oxalic acid, and the like. Inorganic acids are the preferred source of hydrogen ions; with hydrochloric, nitric and phosphoric acids being more preferred and hydrochloric acid being most preferred. An equally acceptable initial treatment involves ion exchange with ammonium salts, such as ammonium chloride. By this method the sodium ions, or their equivalents, are removed, but the aluminum ions are not displaced. On heating the ammonium exchanged mordenite, ammonia is given off and the mordenite is converted to the acid form.

Typically, in the initial acid treatment the original sodium mordenite is slurried with an aqueous solution of the acid. The acid solution may have any concentration, providing the catalyst obtained possesses the properties and activity of the catalyst of this invention, these being described hereinafter. Preferably, the concentration of the aqueous acid solution is in the range from about 0.01N to about 6N; more preferably in the range from about 0.5N to about 3.0N. The relative quantities of aqueous acid solution to mordenite solid which are employed may vary. Typically, the ratio is less than about 15 cc acid solution per gram mordenite solid. Preferably, the ratio is in the range from about 5 cc acid solution per gram mordenite solid to about 10 cc acid solution per gram mordenite solid. The temperature and the duration of the contact of the mordenite with the acid solution may also vary. Preferably, the mordenite is contacted with the acid at a temperature in the range from 10°C to 100°C. Generally, the contact time between the acid solution and the mordenite may vary from 5 minutes to several hours. It is important that there be sufficient time for the acid solution to contact all of the mordenite particles. Preferably, the contact time is from 5 minutes to 60 minutes. The acid extraction, as described herein, may be repeated if desired. Afterwards, the mordenite is washed in water one or more times in order to rinse away soluble species from the mordenite. Preferably, the water wash is carried out at ambient temperature. Optionally, subsequent to the water wash the mordenite is dried in air at a temperature in the range from 20°C to 150°C.

In one treatment, following the exchange with acid and drying in air, the acidic mordenite zeolite is calcined in air or heated in an inert atmosphere, such as nitrogen. It is believed that this heat treatment dislocates a portion of the aluminum from the mordenite framework; however, such a theory should not be taken as limiting of the scope of the invention. Preferably, the temperature of the calcination or heating is in the range from 300°C to 800°C. More preferably, the temperature is in the range from 500°C to 750°C. Most preferably, the temperature is from 650°C to 750°C.

After calcining the acid-treated mordenite described hereinabove, the mordenite is subjected to an additional acid treatment for the purpose of further dealumination. The second acid treatment comprises contacting the calcined mordenite with a strong acid under conditions sufficient to produce the acidic mordenite catalyst of this invention. For the purposes of this invention a "strong" acid is defined as an acid which reacts essentially completely with the solvent to give the conjugate acid of the solvent. For example, if gaseous hydrogen chloride is dissolved in water, the acid-base reaction is complete to give the conjugate acid $H_3O^+$ and $Cl^-$. Preferably, the strong acid is an inorganic acid. More preferably, the strong acid is nitric acid, hydrochloric acid, or sulfuric acid. Most preferably, the strong acid is nitric acid. The concentration of the strong acid will vary depending on the acid selected. In general, the acid is employed in an aqueous solution of any concentration which provides for the extraction of aluminum from the calcined acidic mordenite, as described hereinafter. With nitric acid, for example, the concentration of the acid in the aqueous solution is preferably in the range from about 2N to about 15N. More preferably, the concentration of the acid is in the range from about 4N to about 12N. Most preferably, the concentration of the acid is in the range from about 6N to about 8N. The aqueous acid solution and the calcined mordenite are contacted in any ratio that provides the catalyst of the invention.

Preferably, the ratio of aqueous acid solution to mordenite is in the range from 3 cc acid solution per gram mordenite to 10 cc acid solution per gram mordenite. More preferably, the ratio is about 5 cc acid solution per gram mordenite. The temperature and the duration of the contact may vary depending on the acid selected. Preferably, the mordenite is contacted with the acid solution at a temperature in the range from about ambient temperature taken as 22°C to 220°C. More preferably, the mordenite and the acid are contacted at a temperature which allows for boiling of the aqueous acid under atmospheric conditions. Preferably, the duration of the contact is from hour to 6 hours; more preferably, from 1 hour to 3 hours; most preferably, for about 2 hours. When the contacting with strong acid is complete, the mordenite is filtered and washed repeatedly with water until the washings are acid-free. Preferably, the washed mordenite is heat treated and contacted with strong acid more than once. Lastly, the washed acidic mordenite zeolite is dried for several hours at a temperature in the range from 100°C to 150°C to remove physically adsorbed water. The dried acidic mordenite is activated by heating for about 2 hours at a temperature in the range from 300°C to 700°C. This activation may drive off more strongly bound water and any residual adsorbates.

In an alternative embodiment, the original sodium mordenite is treated with acid and retreated with strong acid without the intermediate calcination step.

The catalyst thus prepared exhibits special characteristics by which it may be identified, specifically, the silica/alumina molar ratio, the Symmetry Index and porosity as defined hereinafter.

As a result of the acid extractions, the silica/alumina molar ratio ($SiO_2/Al_2O_3$) of the acidic mordenite catalyst is increased over that of the original sodium mordenite. Specifically, the acid-treated mordenite catalyst has a silica/alumina molar ratio of higher than 160:1. Preferably, the acid-treated mordenite catalyst has a silica/alumina molar ratio of at least 175:1. More preferably, the silica/alumina molar ratio is at least 190:1. Generally the silica/alumina molar ratio is not higher than 2500:1, more preferably not higher than 1000:1.

As a further result of the acid extractions and, optionally, calcination, the Symmetry Index of the mordenite catalyst is increased over that of the original mordenite. The Symmetry Index is as defined hereinbefore. Since the Symmetry Index is derived from X-ray data, the Index is related to the proportion of Cmcm, and Cmmm, Imcm, or Immm symmetries present in the catalyst. The increase in the Symmetry Index is indicative of the enrichment of the catalyst in the Cmcm component. Preferably, the Symmetry Index ranges 1 to 2.

A third property of the acidic mordenite catalyst, by which it is identified, is the porosity. All mordenites possess pores which form as a natural consequence of mordenite crystal growth. New pores or modifications of existing pores can occur on treating the mordenites, for example, with heat or acid as in the process of this invention. Typically, pores are classified into micropores, mesopores and macropores.

For the purposes of this invention a micropore is defined as having a radius in the range from about 3 Angstrom units (3 Å) to 10 Å. Likewise, a mesopore is defined as having a radius in the range from 10 Å to 100 Å, while a macropore is defined as having a radius from 100 Å to 1000 Å. After calcination and strong acid treatment, the acidic mordenite catalyst of this invention possesses micro-, meso- and macropores. The porosity of the catalyst may be distinguished by the total pore volume defined as the sum of the volumes of the micro-, meso-, and macropores per gram catalyst. A catalyst of this invention has a total pore volume sufficient to provide a high yield of the desired monoalkylated benzene with relatively low levels of polyalkylated products and low levels of impurities. Preferably, the total pore volume is in the range from 0.18 cc/g to 0.45 cc/g. The porosity may be further distinguished by the relative distribution of meso- and macropores, as found in the ratio of the combined meso- and macropore volume to the total pore volume. A catalyst of this invention has a ratio of combined meso- and macropore volume to total pore volume sufficient to provide a high yield of the desired monoalkylated aromatics with relatively low levels of polyalkylated products and low levels of impurities. Preferably, the ratio of the combined meso- and macropore volume to total pore volume is in the range from 0.25 to 0.75.

The measurement of the porosity, described hereinabove, is derived from surface area and pore volume measurements of mordenite powders obtained on any suitable instrument, such as a Quantachrome Digisorb-6 unit, using nitrogen as the adsorbate at the boiling point of nitrogen, 77 K. The total pore volume ($V_T$) is derived from the amount of nitrogen adsorbed at a relative pressure close to unity. It is accepted that this volume constitutes pores of less than 1000 Å in radius. Pores with radius in the 10 Å to 1000 Å range are known in the literature as "transitional pores." The micropore volume ($V_m$) in the presence of "transitional pores" is obtained by the t-method. The difference between the total pore volume and the micropore volume is the transitional pore volume, ($V_t = V_T - V_m$). The cumulative pore distribution in the transitional pore range is used to calculate the relative volume contributions of mesopores and macropores. For example, the mesopore volume is calculated by multiplying the transitional pore volume by the fraction of the cumulative pore volume from 10 Å to 100 Å, ($V_{me} = V_t f_{me}$). The macropore volume is then simply obtained by subtracting the mesopore volume from the transitional volume, ($V_{ma} = V_t - V_{me}$). This approach ensures that the equation $V_T = V_m + V_{me} + V_{ma}$ is satisfied. The adsorption isotherms obtained for the mordenite catalysts of this invention are of Type I, which are described by the Langmuir equation. The Langmuir surface area is obtained from such equation. The methods used to obtain surface areas and pore volumes are described by S. Lowell in Introduction to Powder Surface Area (John Wiley and Sons, 1979), or in the manuals provided with the Digisorb-6 instrument made by the Quantachrome Corporation.

The acidic mordenite catalyst used in the process of the invention is capable of adsorbing biphenyl into the intracrystalline pore system, and conversely desorbing biphenyl. Biphenyl adsorption is effected by exposing the acidic mordenite to biphenyl vapors at 100°C for a time sufficient to obtain near constant weight. Preferably, the adsorption capacity of the acidic mordenite for biphenyl is at least about 5 weight percent. More preferably, the capacity is about 10 weight percent. Biphenyl desorption is effected by heating the mordenite-biphenyl sample in a dynamic helium atmosphere from 25°C to about 1000°C at a heating rate of about 10°C/minute. The desorption of biphenyl may be followed experimentally by thermal gravimetric analysis combined with gas phase chromatography and mass spectrometry (TGA-GC-MS). It is found that weakly adsorbed biphenyl produces a weight loss at temperatures below about 300°C; whereas, strongly adsorbed biphenyl produces a weight loss at temperatures from about 300°C to as high as 1000°C. The amount of strongly adsorbed biphenyl is estimated by subtracting the amount of weakly adsorbed biphenyl from the total amount of biphenyl desorbed from the sample. A fully treated mordenite catalyst of this invention exhibits a sharp weight loss at temperatures below about 300°C, and little or no weight loss from 300°C to 1000°C. In contrast, acid-exchanged mordenite exhibits a sharp weight loss at temperatures below about 300°C, and a second weight loss starting at about 300°C and extending to 1000°C. It is believed that the weakly adsorbed biphenyl is located in sites from which there is relatively easy exit; whereas the strongly adsorbed biphenyl is located in sites from which there is relatively difficult exit. Thus, the acidic mordenite catalyst of this invention provides easy access and egress to adsorbed biphenyl. Such a theory, however, should not be construed to be binding or limiting of the scope of the invention.

The catalysts useful in the process of this invention can be used as such in aggregate form, particle form, or extrudate form. The extrudate form may be obtained, for example, by compressing the aggregates or particles into binderless particles of suitable sizes. Alternatively, the extrudate can be made via use of binders well-known to those in the art.

The catalysts useful in the process of this invention may comprise an inert binder. Preferably, as inert binder silica is used. The binder may comprise between 0 and 90 weight percent of the bound catalyst composition. Preferably, the binder comprises between 5 and 70 weight percent, more preferably between 5 and 40 weight percent.

An additional characteristic of the catalyst is its minimal deactivation in the alkylation of benzene or substituted benzenes. In the process of the present invention, the catalyst remains active for long periods of use, which is quite surprising under the specified conditions.

The catalysts useful in the process of this invention is not sensitive to small amounts of water which may usually be present in benzene or substituted benzene. Accordingly, the benzene or substituted benzene reactant does not have to undergo a drying treatment, which is another advantage of the present process.

In the alkylation reaction of the present invention, the catalyst, should it show any deactivation, may be regenerated by burning off the carbonaceous deposits. This may be effected by leading an oxygen-containing gas over the catalyst at a temperature of 400-700 °C. Preferably a gas stream containing between about 1 and 30 percent oxygen is passed over the catalyst at 500-600 °C.

The ratio of the benzene or substituted benzene to catalyst may be any weight ratio which produces the desired monoalkylated benzene with a relatively high selectivity the dialkylated benzene being the major byproduct, whereas low level of tri- and tetra- or higher polyalkylated products are achieved as well as a low level of other impurities. Preferred ratios will also be dependent on the way the process is operated. For example, in a continuous mode of operation the weight hourly space velocity (WHSV) of the overall feed with respect to catalyst is preferably in the range from 0.5 to 100.

More preferably, the WHSV is in the range from 0.5 to 20.

The molar ratio of benzene or substituted benzene to alkylating agent is less than 3:1 and greater than 1:1. Preferably, the ratio of benzene or substituted benzene to alkylating agent is between 1.7:1 and 2.9:1, more preferably between 2.0:1 and 2.7:1.

The benzene or substituted benzene and alkylating agent may be introduced to the reactor or reaction zone as separate feeds or as combined feeds. Further, either the benzene or substituted benzene and the alkylating agent may be introduced to the reactor or reaction zone as a single feed stream or split into a plurality of feed streams which are introduced into the reactor at different locations. In the latter case, at least one of the subsequent streams is introduced into the main feed stream after the reactants therein have at least partially reacted. For example, in a reactor containing one or more fixed catalyst beds, a subsequent stream is introduced after the main stream has passed at least one catalyst bed or a portion thereof, and before the main stream enters a further catalyst bed or a portion thereof. In the process of the present invention preferably a plurality of catalyst containing reaction zones in fluid connection in series is used, wherein the whole of the benzene or substituted benzene is delivered to a first reaction zone, and a series of fractions of alkylating agent are delivered respectively to the first reaction zone and between each pair of contiguous reaction zones. The reaction zones may be operated with the same or different alkylation catalyst and at the same or different temperature and WHSV. More preferably, two to twenty catalyst containing reaction zones are used and a corresponding number of alkylating agent streams. Operating the process in this way has been found to increase the selectivity towards the monoalkylated product, compared to supplying the whole of the alkylating agent to the (first) reaction zone. Furthermore, by feeding the alkylating agent as a series of fractions, a better control of the reaction temperature is possible for this exothermic reaction. Alternatively or additionally, the reaction zone(s) may be cooled by conventional means.

The contacting of the benzene or substituted benzene with the alkylating agent in the presence of the catalyst may occur in a reactor of any configuration. Batch-type and continuous reactors, such as fixed bed, slurry bed, fluidized bed, catalytic distillation, or countercurrent reactors, are suitable configurations for the contact. Preferably, the reactor is a continuous reactor.

The process of the invention is carried out under conditions sufficient to keep the reaction mixture in the liquid phase. This means that substantially no gaseous zone is present in the reactor. With substantially no gaseous zone is meant, that the gaseous zone comprises at the most 5 percent by volume of the reaction zone, preferably at the most 1 percent by volume. Most preferably, the reactor is operated in a liquid full mode. The presence of a substantial gaseous zone could lead to an accumulation of the alkylating agent therein, which under the prevailing conditions would polymerize. This in its turn would lead to a decrease in selectivity and to an increase in deactivation of the catalyst. The benzene or substituted benzene may be in the molten, liquid form or in solution. The alkylating agent may be introduced in the liquid or gaseous state, and should substantially dissolve in the liquid phase. The catalyst may be used in various forms, such as a fixed bed, moving bed, fluidized bed, in suspension in the liquid reaction mixture, or in a reactive distillation column.

The contacting of the reactants in the presence of the catalyst may occur at any temperature and pressure conditions sufficient to keep the reaction mixture in the liquid phase. Typically, the temperature is in the range from 100°C to 300°C. These temperatures are relatively mild for zeolite catalyzed type alkylation processes. Below this lower limit of 100°C the reaction proceeds slowly. In one preferred mode of the present process benzene is contacted with propylene as alkylating agent and the temperature is in the range from 120°C to 250°C. In another preferred mode benzene is contacted with ethylene as alkylating agent and the temperature is in the range from 170°C to 280°C.

The pressure in the reactor may be any pressure sufficient to keep the reaction mixture as a liquid under reaction conditions. The required pressure will change depending on temperature and reactants employed. Preferably, the pressure in the reactor is in the range from about 10 bar to about 200 bar. More preferably, the pressure is in the range from about 20 bar to about 100 bar.

Following the alkylation of the benzene or substituted benzene, the product mixture may be separated by standard techniques. The desired monoalkylated products, such as for example cumene or ethylbenzene, are separated from the starting materials and byproducts by techniques known in the art such as distillation. Normally, when using a distillation sequence as separation technique, the benzene or substituted benzene starting material may be obtained as a first fraction, which may partially be recycled to the alkylation step and/or partially be fed to an optional subsequent transalkylation step. As a second fraction in the distillation sequence substantially pure monoalkylated product may be recovered. A third fraction may be separated which contains polyalkylated benzene or polyalkylated substituted benzene products and heavies and by-products like for example diphenyl alkanes. Alternatively and preferably a third fraction is collected containing primarily dialkylated products and optionally tri- and/or tetra-alkylated products. This latter third fraction, preferably containing no tetra-alkylated products, may be fed to a subsequent transalkylation step.

According to a further aspect of the invention, a polyalkylated benzene or polyalkylated substituted benzene fraction separated from the reaction product of the alkylation process as described hereinbefore is subjected to a transalkylation step by contacting the polyalkylated benzene or polyalkylated substituted benzene fraction with benzene or substituted benzene in the presence of a suitable transalkylation catalyst to produce monoalkylated benzene or monoalkylated substituted benzene.

The polyalkylated benzene or polyalkylated substituted benzene fraction obtained in the alkylation process of the present invention comprises a major portion of the dialkylated product and minor portions of the tri- and optionally but less preferably tetra- and higher polyalkylated products. As dialkylated products generally act as transalkylating agent under milder conditions than the higher-alkylated products this fraction is a very suitable transalkylating agent for the benzene or substituted benzene in the transalkylation step.

In the transalkylating step the polyalkylated benzene or polyalkylated substituted benzene fraction preferably is contacted with benzene or substituted benzene in the presence of a catalyst comprising an acidic mordenite zeolite having a silica/alumina molar ratio of at least 50:1. This transalkylation catalyst may be any prior art acidic mordenite zeolite having a silica/alumina molar ratio of at least 50:1 and having transalkylation activity.

It is preferred, however, that the catalyst used in the transalkylating step additionally has a crystalline structure which is determined by X-ray diffraction to have a Symmetry Index of at least 1. The overall selectivity of monoalkylated product achieved in the alkylation-transalkylation process of the present invention, based on the benzene or substituted benzene used, is very high, preferably at least 97 mole percent and more preferably at least 99 mole percent. Thus an overall process is obtained having a relatively low amount of recycled benzene or substituted benzene, combined with substantially 100 percent conversion of the alkylating agent and a very high selectivity of monoalkylated product. Preferably, the catalyst used in the transalkylating step has a Symmetry Index of between 1 and 2. This transalkylation catalyst shows little or no deactivation and is capable of converting compounds like diphenyl alkanes, such as for example diphenyl ethane, which are produced in the alkylation process as undesired byproducts, to the desired monoalkylated products, optionally in a separate diphenyl alkane conversion step. It is surprising that also diphenyl alkane is converted to monoalkylated product and benzene as this is no transalkylation in the usual meaning. Different from a transalkylation reaction, this conversion requires an additional hydrogen atom.

It is preferred that the catalyst used in the transalkylating step has a silica/alumina molar ratio of between 50:1 and about 500:1. The transalkylation catalyst may comprise an inert binder. Binders known to be useful with mordenite zeolite catalysts, such as for example silica, are useful for this purpose. The binder may comprise between about 0 and 90 weight percent of the bound catalyst composition. Preferably, the binder comprises between about 5 and 70 weight percent, more preferably between about 5 and 40 weight percent. In a most preferred embodiment, the binder is silica.

In a continuous mode of operation the WHSV of the transalkylation feed with respect to catalyst is preferably in the range from 0.1 to 100. More preferably, the WHSV is in the range from 0.1 to 20.

In the transalkylating step the molar ratio of the total of benzene groups, present in the benzene or substituted benzene and in the polyalkylated benzene or polyalkylated substituted benzene fraction, to the total of alkylated groups on the polyalkylated benzene or polyalkylated substituted benzene is preferably at least 1.5:1. In case of a lower ratio the selectivity to the monoalkylated product is decreased. More preferably the molar ratio is from 2:1 to 4:1.

The benzene or substituted benzene and transalkylating agent may be introduced to the reactor or reaction zone as separate feeds or as combined feeds. Further, either the benzene or substituted benzene and the transalkylating agent may be introduced to the reactor or reaction zone as a single feed stream or split into a plurality of feed streams which are introduced into the reactor at different locations. In the transalkylation process of the present invention preferably a plurality of catalyst containing reaction zones in fluid connection in series is used, wherein the whole of the benzene or substituted benzene is delivered to a first reaction zone, and a series of fractions of transalkylating agent are delivered respectively to the first reaction zone and between each pair of contiguous reaction zones. Operating the process in this way would increase the selectivity towards the monoalkylated product compared to supplying the whole of the transalkylating agent to the (first) transalkylation reaction zone.

The contacting of the benzene or substituted benzene with the transalkylating agent in the presence of the catalyst may occur in a reactor of any configuration. Batch-type and continuous reactors, such as fixed bed, slurry bed, fluidized bed, catalytic distillation, or countercurrent reactors, are suitable configurations for the contact. Preferably, the reactor is a continuous flow reactor.

The transalkylation step of the invention is preferably carried out under conditions sufficient to keep the reaction mixture in the liquid phase. Most preferably, the reactor is operated in a substantially liquid full mode. The benzene or substituted benzene and the transalkylating agent may be in the molten, liquid form or in solution. The catalyst may be used in various forms, such as a fixed bed, moving bed, fluidized bed, in suspension in the liquid reaction mixture, or in a reactive distillation column.

The contacting of the reactants in the presence of the catalyst may occur at any temperature and pressure conditions sufficient to keep the reaction mixture in the liquid phase. Typically, in the transalkylation the temperature is in the range from 140°C to 300°C. These temperatures are relatively mild for zeolite catalyzed type transalkylation processes. In one preferred mode of the present transalkylation step a polyisopropylated benzene fraction as the polyalkylated benzene fraction is contacted with benzene and the temperature is in the range from 140°C to 250°C. In another preferred mode a polyethylated benzene fraction as the polyalkylated benzene fraction is contacted with benzene and the temperature is in the range from 200°C to 300°C.

The pressure in the reactor may be any pressure sufficient to keep the reaction mixture as a liquid under reaction conditions. The required pressure will change depending on temperature and reactants employed. Preferably, the pressure in the reactor is in the range from 10 bar to 200 bar. More preferably, the pressure is in the range from 20 bar to 100 bar.

The transalkylation step, when performed in the presence of the preferred transalkylation catalyst, does not require the addition of hydrogen to the transalkylation feed. Prior art transalkylation processes are frequently carried out in the presence of hydrogen in order to remove carbonaceous deposits from the catalyst in order to limit deactivation thereof. In view of the minimal deactivation of the preferred transalkylation catalyst in the present process, no addition of hydrogen is required.

The catalyst, should it show any deactivation after extensive periods of use, may be regenerated by burning off the carbonaceous deposits. This may be effected by leading an oxygen-containing gas over the catalyst at a temperature of 400-700°C. Preferably a gas stream containing between about 1 and 30 percent oxygen is passed over the catalyst at 500-600°C.

An additional factor that is important is the presence of various impurities in the product. Even very small amounts of certain impurities such as n-propylbenzene or propylene oligomers in the case of cumene, or xylenes in the case of ethylbenzene, create significant problems in various applications. Processes run under different conditions result in different levels of impurities. Thus, a particular advantage of the overall alkylation-transalkylation process of the present invention is the low impurity levels. In the case of cumene production, low levels of oligomers as indicated by low bromine index is also important. In cumene production, the bromine index is preferably no greater than about 100, more preferably no greater than about 50 and most preferably no greater than about 20. Cumene produced by the process of this invention preferably contains less than about 1000 parts per million (ppm) impurities, more preferably less than about 200 ppm. Ethylbenzene produced by the process of this invention preferably has less than about 200 ppm xylene impurities, more preferably less than about 100 ppm.

### Specific Embodiments

The following examples are given to illustrate the catalyst and the process of this invention and should not be construed as limiting its scope. All percentages in the examples are mole percent unless otherwise indicated. In all the experiments a continuous flow fixed catalyst bed type reactor was used.

### Example 1 - Catalyst Preparation

Alkylation catalysts A-1 and A-2 are prepared from Na-mordenite having a $SiO_2/Al_2O_3$ ratio of 19 and a Symmetry Index of about 1.2 according to substantially the same procedure. The Na-mordenite is ion-exchanged to remove sodium, then calcined. The product is leached with HCl to give a H-mordenite having a $SiO_2/Al_2O_3$ ratio of about 220 (catalyst A-1) and 196 (catalyst A-2). The H-mordenite is next pelletized with 20 percent silica binder. Finally, the pellets are calcined at 750°C. The other characteristics of the catalysts are: catalyst A-1: Symmetry Index of 1.85; BET of 389 $m^2/g$; total pore volume of 0.374 ml/g; micro pore volume of 0.156 ml/g; meso pore volume of 0.140 ml/g; macro pore volume of 0.078 ml/g; and meso + macro pore volume/ total pore volume of 0.58. For catalyst A-2: Symmetry Index of 1.98; BET of 392 $m^2/g$; total pore volume of 0.384 ml/g; micro pore volume of 0.158 ml/g; meso pore volume of 0.135 ml/g; macro pore volume of 0.091 ml/g; and meso + macro pore volume/ total pore volume of 0.59.

Transalkylation catalyst T-1 is prepared in a procedure similar to that of Catalyst A-1 using a starting sodium mordenite having a $SiO_2/Al_2O_3$ ratio of about 15 and a Symmetry Index of about 1.0. It is exchanged with 1.0 N HCl to remove sodium and subsequently washed and calcined at 500°C for 2 hours. Then it is extracted under reflux conditions with 6 N HCl for 2 hours, washed and dried at 110°C, to give a $SiO_2/Al_2O_3$ ratio of about 81. This dealuminated mordenite is then mixed with 20 weight percent silica binder and calcined at 700°C to give a catalyst with a Symmetry Index of 1.42; a benzene adsorption capacity of 13.7 percent (w/w); a BET of 382 $m^2/g$; a total pore volume of 0.341 ml/g; a macro pore volume of 0.156 ml/g; and a meso + macro pore volume/ total pore volume of 0.54.

Catalysts A-1, A-2 and T-1 are determined by X-ray diffraction to have Cmcm symmetry having dispersed therein domains of Cmmm symmetry.

### Example 2: Alkylation of Benzene with Ethylene

A feed stream of benzene and ethylene having a benzene to ethylene mole ratio of 2.22 is subjected to alkylation over catalyst A-2 at 250°C and a pressure of 36 bar. The WHSV of the feed stream is about 1. The reactor effluent contains 24.9 weight percent ethylbenzene, 58.0 weight percent benzene, 13.5 weight percent diethylbenzene (m-diethylbenzene, 8.48 percent; p-diethylbenzene 4.06; o-diethylbenzene, 0.94 percent), and 3.30 weight percent triethylbenzene. No xylenes are observed. The ethylene conversion is 100 percent. The benzene conversion is 31 percent. The catalyst has high stability after 250 continuous hours of operation.

### Example 3: Alkylation of Benzene with Ethylene

A feed stream of benzene and ethylene having an overall benzene to ethylene ratio of 2.27 is subjected to alkylation over catalyst A-1 at 220°C and a pressure of 50 bar. The WHSV of the feed stream is about 1. The ethylene feed is split in two parts: 50 percent of the ethylene is fed to the reactor inlet and 50 percent is introduced at half of the catalyst bed. The conversion of ethylene is 100 percent. The conversion of benzene is 33 percent. The reactor effluent contains 27.3 wt percent ethylbenzene, 57.9 wt percent benzene, 12.6 wt percent ortho- + meta- + para- diethylbenzenes, 1.8 wt percent triethylbenzene and 0.05 wt percent tetraethylbenzenes. No xylenes are observed. The catalyst has a high stability. The catalyst shows essentially no deactivation after over 600 hours of continuous use.

Example 4: Alkylation of Benzene with Ethylene

A feed stream of benzene and ethylene having an overall benzene to ethylene ratio of 2.63 is subjected to alkylation over catalyst A-1 at 240°C and a pressure of 50 bar. The WHSV of the feed stream is about 2. The ethylene feed is split in two parts: 50 percent of the ethylene is fed to the reactor inlet and 50 percent at half of the catalyst bed. Conversion of ethylene is 100 percent. The conversion of benzene is 31 percent. The reactor effluent contains 26.1 wt percent ethylbenzene, 61.6 wt percent benzene, 10.6 wt percent ortho- + meta- + para-diethylbenzene, 1.4 wt percent triethylbenzene and 0.04 wt percent tetraethylbenzene. No xylenes are observed. The catalyst shows essentially no deactivation after over 500 hours of continuous use.

Example 5: Transalkylation of Benzene with Polyethylbenzenes

A feedstream of 65.2 wt percent benzene, 29.8 wt percent meta + ortho + para- diethylbenzene, 1.5 wt percent triethylbenzenes and 2.5 wt percent ethylbenzene having an overall benzene groups to ethylene groups mole ratio of 2.2 is subjected to transalkylation over catalyst T-1 at 235°C and a pressure of 36 bar. The WHSV of the feed stream is about 1. The catalyst has a very high stability and shows essentially no deactivation after 3000 hours of continuous use. The conversion of diethylbenzenes is 68 percent. The conversion of triethylbenzenes is 15 percent. The selectivity of polyethylbenzenes to ethylbenzene is 97 percent.

Example 6: Transalkylation of Benzene with Polyethylbenzene

A feedstream of 69.6 wt percent benzene, 26.5 wt percent meta- + ortho- + para-ethylbenzene, 1.4 wt percent triethylbenzene and 2.5 wt percent ethylbenzene having an overall benzene groups to ethylene groups mole ratio of 2.5 is subjected to transalkylation over catalyst T-1 at 260°C and a pressure of 36 bar. The WHSV of the feedstream is 2.7. The effluent contains 32.2 wt percent ethylbenzene. Conversion of the diethylbenzenes is 74 percent. The catalyst shows no deactivation after 500 hours of continuous use.

**Claims**

1. A process of alkylating benzene or substituted benzene comprising contacting the benzene or substituted benzene with an alkylating agent having from two to eighteen carbon atoms in the presence of a catalyst comprising an acidic mordenite zeolite having a silica/alumina molar ratio of higher than 160:1 and a crystalline structure which is determined by X-ray diffraction to have a Symmetry Index of at least 1, under temperature and pressure conditions sufficient to keep the reaction mixture in the liquid phase, and the molar ratio of benzene or substituted benzene to alkylating agent is less than 3:1 and greater than 1:1.

2. The process of Claim 1 wherein the catalyst has a silica/alumina molar ratio of at least 175:1.

3. The process of Claims 1-2 wherein the catalyst has a Symmetry Index from 1 to 2.

4. The process of Claims 1-3 wherein the catalyst comprises an inert binder.

5. The process of Claim 4 wherein the inert binder is silica binder.

6. The process of Claims 1-5 wherein the molar ratio of benzene or substituted benzene to alkylating agent is between 1.7:1 and 2.9:1.

7. The process of Claim 6 wherein the molar ratio of benzene or substituted benzene to alkylating agent is between 2.0:1 and 2.7:1.

8. The process of Claims 1-7 wherein a plurality of catalyst containing reaction zones in fluid connection in series is used, wherein the whole of the benzene or substituted benzene is delivered to a first reaction zone, and a series of fractions of alkylating agent are delivered respectively to the first reaction zone and between each pair of contiguous reaction zones.

9. The process of Claim 8 wherein two to twenty catalyst containing reaction zones are used and a corresponding number of alkylating agent streams.

10. The process of Claims 1-9 wherein the temperature is in the range from 100°C to 300°C.

11. The process of Claims 1-10 wherein benzene is contacted with propylene as alkylating agent and the temperature is in the range from 120°C to 250°C.

12. The process of Claims 1-10 wherein benzene is contacted with ethylene as alkylating agent and the temperature is in the range from 170°C to 280°C.

13. The process of Claims 1-12 wherein the pressure is in the range from 10 bar to 200 bar.

14. The process of Claim 13 wherein the pressure is in the range from 20 bar to 100 bar.

15. The process of Claims 1-14 wherein a polyalkylated benzene or polyalkylated substituted benzene fraction separated from the reaction product of the alkylation process of Claims 1-14 is subjected to a transalkylation step by contacting the polyalkylated benzene or polyalkylated substituted benzene fraction with benzene or substituted benzene in the presence of a suitable transalkylation catalyst to produce monoalkylated benzene or monoalkylated substituted benzene.

16. The process of Claim 15 wherein in the transalkylating step the polyalkylated benzene or polyalkylated substituted benzene fraction is contacted with benzene or substituted benzene in the presence of a catalyst comprising an acidic mordenite zeolite having a silica/alumina molar ratio of at least 50:1.

17. The process of Claims 16 wherein the catalyst used in the transalkylating step has a crystalline structure which is determined by X-ray diffraction to have a Symmetry Index of at least 1.

18. The process of Claim 17 wherein the catalyst used in the transalkylating step has a Symmetry Index of between 1 and 2.

19. The process of Claim 16-18 wherein the catalyst used in the transalkylating step has a silica/alumina molar ratio of between 50:1 and 500:1.

20. The process of Claims 16-19 wherein the catalyst used in the transalkylating step comprises an inert binder.

21. The process of Claim 20 wherein the inert binder is silica.

22. The process of Claims 16-21 wherein in the transalkylating step the molar ratio of the total of benzene groups, present in the benzene or substituted benzene and in the polyalkylated benzene or polyalkylated substituted benzene fraction, to the total of alkylated groups on the polyalkylated benzene or polyalkylated substituted benzene is at least 1.5:1.

23. The process of Claims 16-22 wherein in the transalkylating step the temperature is in the range from 140°C to 300°C and the pressure is sufficient to keep the reaction mixture in the liquid phase.

24. The process of Claims 16-23 wherein in the transalkylating step a polyisopropylated benzene fraction as the polyalkylated benzene fraction is contacted with benzene and the temperature is in the range from 140°C to 250°C.

25. The process of Claims 16-23 wherein in the transalkylating step a polyethylated benzene fraction as the polyalkylated benzene fraction is contacted with benzene and the temperature is in the range from 200°C to 300°C.

**Patentansprüche**

1. Verfahren zum Alkylieren von Benzol oder substituiertem Benzol, umfassend das Kontaktieren des Benzols oder substituierten Benzols mit einem Alkylierungsmittel, das 2 bis 18 Kohlenstoffatome aufweist, in der Gegenwart eines Katalysators, umfassend einen sauren Mordenitzeolith, mit einem molaren Verhältnis von Siliciumdioxid/Aluminiumoxid von größer als 160:1 und einer Kristallstruktur, von der durch Röntgenstrahldiffraktion bestimmt wurde, daß sie einen Symmetrieindex von mindestens 1 aufweist, unter Temperatur- und Druckbedingungen, die ausreichend sind, um das Reaktionsgemisch in der flüssigen Phase zu halten und worin das molare Verhältnis von Benzol oder substituiertem Benzol zu Alkylierungsmittel kleiner als 3:1 und größer 1:1 ist.

2. Verfahren nach Anspruch 1, worin der Katalysator ein molares Verhältnis von Siliciumdioxid/Aluminiumoxid von mindestens 175:1 hat.

3. Verfahren nach Anspruch 1 bis 2, worin der Katalysator einen Symmetrieindex von 1 bis 2 hat.

4. Verfahren nach Anspruch 1 bis 3, worin der Katalysator ein inertes Bindemittel umfaßt.

5. Verfahren nach Anspruch 4, worin das inerte Bindemittel ein Siliciumdioxidbindemittel ist.

6. Verfahren nach Anspruch 1 bis 5, worin das molare Verhältnis von Benzol oder substituiertem Benzol zu Alkylierungsmittel zwischen 1,7:1 und 2,9:1 ist.

7. Verfahren nach Anspruch 6, worin das molare Verhältnis von Benzol oder substituiertem Benzol zu Alkylierungsmittel zwischen 2,0:1 und 2,7:1 ist.

8. Verfahren nach Anspruch 1 bis 7, worin mehrere Katalysator enthaltende Reaktionszonen in Serie in Fluidverbindung verwendet wird, worin das gesamte Benzol oder substituierte Benzol in eine erste Reaktionszone eingebracht wird und eine Alkylierungsmittelfraktionsserie jeweils in die erste Reaktionszone und zwischen jedes Paar von aufeinanderfolgenden Reaktionszonen eingebracht wird.

9. Verfahren nach Anspruch 8, worin 2 bis 20 Katalysator enthaltende Reaktionszonen und eine entsprechende Anzahl Alkylierungsmittelströme verwendet werden.

10. Verfahren nach Anspruch 1 bis 9, worin die Temperatur im Bereich von 100°C bis 300°C ist.

11. Verfahren nach Anspruch 1 bis 10, worin Benzol mit Propylen als Alkylierungsmittel kontaktiert wird und die Temperatur im Bereich von 120°C bis 250°C ist.

12. Verfahren nach Anspruch 1 bis 10, worin Benzol mit Ethylen als Alkylierungsmittel kontaktiert wird und die Temperatur im Bereich von 170°C bis 280°C ist.

13. Verfahren nach Anspruch 1 bis 12, worin der Druck im Bereich von 10 bar bis 200 bar ist.

14. Verfahren nach Anspruch 13, worin der Druck im Bereich von 20 bar bis 100 bar ist.

15. Verfahren nach Anspruch 1 bis 14, worin eine Fraktion aus polyalkyliertem Benzol oder polyalkyliertem substituiertem Benzol getrennt vom Reaktionsprodukt des Alkylierungsverfahrens von Anspruch 1 bis 14 einem Transalkylierungsschritt unterworfen wird durch Kontaktieren der Fraktion aus polyalkyliertem Benzol oder polyalkyliertem substituiertem Benzol mit Benzol oder substituiertem Benzol in der Gegenwart eines geeigneten Transalkylierungskatalysators, um monoalkyliertes Benzol oder monoalkyliertes substituiertes Benzol zu bilden.

16. Verfahren nach Anspruch 15, worin im Transalkylierungsschritt die Fraktion aus polyalkyliertem Benzol oder polyalkyliertem substituiertem Benzol mit Benzol oder substituiertem Benzol in der Gegenwart eines Katalysators, umfassend einen sauren Mordenitzeolith mit einem molaren Verhältnis von Siliciumdioxid/Aluminiumoxid von mindestens 50:1, kontaktiert wird.

17. Verfahren nach Anspruch 16, worin der im Transalkylierungsschritt verwendete Katalysator eine Kristallstruktur aufweist, die, bestimmt durch Röntgenstrahldiffraktion, einen Symmetrieindex von mindestens 1 aufweist.

18. Verfahren nach Anspruch 17, worin der im Transalkylierungsschritt verwendete Katalysator einen Symmetrioindex zwischen 1 und 2 aufweist.

19. Verfahren nach Anspruch 16 bis 18, worin der im Transalkylierungsschritt verwendete Katalysator ein molares Verhältnis von Siliciumdioxid/Aluminiumoxid von zwischen 50:1 und 500:1 aufweist.

20. Verfahren nach Anspruch 16 bis 19, worin der im Transalkylierungsschritt verwendete Katalysator ein inertes Bindemittel umfaßt.

21. Verfahren nach Anspruch 20, worin das inerte Bindemittel Siliciumdioxid ist.

22. Verfahren nach Anspruch 16 bis 21, worin im Transalkylierungsschritt das molare Verhältnis der gesamten Benzolgruppen, die im Benzol oder substituierten Benzol und in der Fraktion aus polyalkyliertem Benzol oder polyalkyliertem substituiertem Benzol vorliegen zu den gesamten alkylierten Gruppen des polyalkylierten Benzols oder polyalkylierten substituierten Benzols mindestens 1,5:1 ist.

23. Verfahren nach Anspruch 16 bis 22, worin im Transalkylierungsschritt die Temperatur im Bereich von 140°C bis 300°C ist und der Druck ausreichend ist, um das Reaktionsgemisch in der flüssigen Phase zu halten.

24. Verfahren nach Anspruch 16 bis 23, worin im Transalkylierungsschritt eine Fraktion aus polyisopropyliertem Benzol als die Fraktion aus polyalkyliertem Benzol, mit Benzol kontaktiert wird und die Temperatur im Bereich von 140°C bis 250°C ist.

25. Verfahren nach Anspruch 16 bis 23, worin im Transalkylierungsschritt eine Fraktion aus polyethyliertem Benzol als die Fraktion aus polyalkyliertem Benzol mit Benzol kontaktiert wird und die Temperatur im Bereich von 200°C bis 300°C ist.

**Revendications**

1. Procédé d'alkylation du benzène ou de benzène substitué consistant à mettre en contact le benzène ou le benzène substitué avec un agent d'alkylation ayant de deux à dix-huit atomes de carbone en présence d'un catalyseur comprenant une zéolite de mordénite acide ayant un rapport molaire silice/alumine supérieur à 160:1 et une structure cristalline, telle que déterminée par diffraction des rayons X, ayant un indice de symétrie d'au moins 1, dans des conditions de température et de pression suffisantes pour maintenir le mélange réactionnel en phase liquide, le rapport molaire de benzène ou de benzène substitué à l'agent d'alkylation étant inférieur à 3:1 et supérieur à 1:1.

2. Procédé selon la revendication 1, dans lequel le catalyseur possède un rapport molaire silice/alumine d'au moins 175:1.

3. Procédé selon l'une des revendications 1 ou 2, dans lequel le catalyseur possède un indice de symétrie de 1 à 2.

4. Procédé selon l'une des revendications 1 à 3, dans lequel le catalyseur comprend un liant inerte.

5. Procédé selon la revendication 4, dans lequel le liant inerte est un liant à base de silice.

6. Procédé selon l'une des revendications 1 à 5, dans lequel le rapport molaire de benzène ou de benzène substitué à l'agent d'alkylation est compris entre 1,7:1 et 2,9:1.

7. Procédé selon la revendication 6, dans lequel le rapport molaire de benzène ou de benzène substitué à l'agent d'alkylation est compris entre 2,0:1 et 2,7:1.

8. Procédé selon l'une des revendications 1 à 7, dans lequel on utilise un ensemble de zones réactionnelles contenant un catalyseur en connexion fluide en série, dans lequel la totalité du benzène ou du benzène substitué est délivrée à une première zone réactionnelle, et une série de fractions d'agent d'alkylation est délivrée respectivement à la première zone de réaction et entre chaque paire de zones réactionnelles contiguës.

9. Procédé selon la revendication 8, dans lequel deux à vingt zones réactionnelles contenant un catalyseur et un nombre correspondant de courants d'agent d'alkylation sont utilisées.

10. Procédé selon l'une des revendications 1 à 9, dans lequel la température est comprise entre 100°C et 300°C.

11. Procédé selon l'une des revendications 1 à 10, dans lequel le benzène est mis en contact avec du propylène en tant qu'agent d'alkylation et la température est comprise entre 120°C et 250°C.

12. Procédé selon l'une des revendications 1 à 10, dans lequel le benzène est mis en contact avec de l'éthylène en tant qu'agent d'alkylation et la température est comprise entre 170°C et 280°C.

13. Procédé selon l'une des revendications 1 à 12, dans lequel la pression est comprise entre 10 bars et 200 bars.

14. Procédé selon la revendication 13, dans lequel la pression est comprise entre 20 bars et 100 bars.

15. Procédé selon l'une des revendications 1 a 14, dans lequel une fraction du benzène polyalkylé ou du benzène substitué polyalkylé séparée du produit réactionnel du procédé d'alkylation selon l'une des revendications 1 à 14, est soumise à une étape de transalkylation en mettant en contact la fraction de benzène polyalkylé ou de benzène substitué polyalkylé avec du benzène ou du benzène substitué en présence d'un catalyseur de transalkylation approprié pour produire un benzène monoalkylé ou un benzène substitué monoalkylé.

16. Procédé selon la revendication 15, dans lequel, dans l'étape de transalkylation, la fraction de benzène polyalkylé ou de benzène substitué polyalkylé est mise en contact avec du benzène ou du benzène substitué en présence d'un catalyseur comprenant une zéolite de mordénite acide ayant un rapport molaire silice/alumine d'au moins 50:1.

17. Procédé selon la revendication 16, dans lequel le catalyseur utilisé dans l'étape de transalkylation possède une structure cristalline, telle que déterminée par diffraction des rayons X, ayant un indice de symétrie d'au moins 1.

**18.** Procédé selon la revendication 17, dans lequel le catalyseur utilisé dans l'étape de transalkylation possède un indice de symétrie compris entre 1 et 2.

**19.** Procédé selon l'une des revendications 16 à 18, dans lequel le catalyseur utilisé dans l'étape de transalkylation possède un rapport molaire silice/alumine compris entre 50:1 et 500:1.

**20.** Procédé selon l'une des revendications 16 à 19, dans lequel le catalyseur utilisé dans l'étape de transalkylation comprend un liant inerte.

**21.** Procédé selon la revendication 20, dans lequel le liant inerte est la silice.

**22.** Procédé selon l'une des revendications 16 à 21, dans lequel, dans l'étape de transalkylation, le rapport molaire de la totalité des groupes benzène, présents dans le benzène ou le benzène substitué et dans la fraction de benzène polyalkylé ou de benzène substitué polyalkylé, à la totalité des groupes alkylés sur le benzène polyalkylé ou le benzène substitué polyalkylé est d'au moins 1,5:1.

**23.** Procédé selon l'une des revendications 16 à 22, dans lequel, dans l'étape de transalkylation, la température est comprise entre 140°C et 300°C et la pression est suffisante pour maintenir le mélange réactionnel en phase liquide.

**24.** Procédé selon l'une des revendications 16 à 23, dans lequel, dans l'étape de transalkylation, une fraction de benzène polyisopropylé, en tant que fraction de benzène polyalkylé, est mise en contact avec du benzène et la température est comprise entre 140°C et 250°C.

**25.** Procédé selon l'une des revendications 16 à 23, dans lequel, dans l'étape de transalkylation, on met en contact une fraction de benzène polyéthylé, en tant que fraction de benzène polyalkylé, avec du benzène et la température est comprise entre 200°C et 300°C.